# EUROPEAN PATENT APPLICATION

(11) **EP 4 671 716 A1**
(43) Date of publication of application: **31.12.2025**
(21) Application number: 24306021.7
(22) Date of filing: 25.06.2024
(51) Int. Cl.: G01L 1/22, G01L 5/1627, A61B 90/00

(54) **DEVICE MEASURING THE FORCE AT A HERNIA MESH FIXATION**

(71) Applicant: Sofradim Production, 01600 Trevoux (FR)
(72) Inventor: LE RUYET, Anicet, 01600 Trevoux (FR); BAILLY, Pierre, 01600 TREVOUX (FR); VEGLEUR, Anthony, 01600 Trevoux (FR); JOURDAN, Arthur, 01600 Trevoux (FR)
(74) Representative: Germain Maureau

(57) **Abstract**

This document discloses system and method embodiments for measuring shear forces at fixation points of textile-based implants. A system for supporting tissue includes two or more sensor assemblies (110), each sensor assembly (110) including a base portion (116) configured to be anchored in tissue (120), a beam (114) extending away from the base portion (116) toward an attachment surface (112), and a sensor (130) attached to the beam (114) and configured to measure a force applied to the beam (114). The system further includes a mesh (102) configured to be disposed adjacent to the tissue (120), the mesh (102) configured to attach to the attachment surface (112) of each of the sensor assemblies (110) such that mesh tension applies a shear force to the attachment surface (112) of a sensor assembly (110). The system further includes readout electronics configured to receive force information from each sensor assembly (110).

## Description

### FIELD

The present disclosure relates to textile-based implants and, more particularly, to systems, devices, and methods for measuring shear forces at fixation points of textile-based implants.

### BACKGROUND

Most hernias are caused by a combination of pressure and an opening or weakness of muscle or connective tissue. The pressure may force tissue or part of an organ through the opening or weak spot. A surgical mesh is a medical device that is used to provide additional support to weakened or damaged tissue. Various types of synthetic or biologic material may be woven or knitted into a mesh and used to reinforce the repair of hernias. Surgical mesh made of synthetic materials can be found in knitted mesh or non-knitted sheet forms. The synthetic materials used can be absorbable, non-absorbable or a combination of absorbable and non-absorbable materials. General surgeons secure the mesh over the defect (e.g., hernia site) using sutures, adhesive, staples, or tacks. Fixation can be absorbable or non-absorbable. Following the surgery, the mesh becomes integrated in the abdominal wall as new tissue grows and remodels due to the porous structure of the implant, strengthening the abdominal/body wall.

After implantation, and depending on its material properties such as strength or stiffness, the mesh is subjected to static and/or cyclic loading, potentially leading to stress concentration, generally in the form of shear forces, at the points of attachment. This can lead to 1) acute pain 2) risk of mesh failure, potentially leading to hernia recurrence. Furthermore, these issues may arise without prior warning of undue forces at the points of attachment.

This document describes methods and systems that are directed to addressing the problems described above, and/or other issues.

### SUMMARY

The techniques of this disclosure generally relate to systems, devices, and methods for measuring shear forces at fixation points of textile-based implants. Issues associated with prior solutions are addressed by the subject matter of the independent claims included in this document. Additional advantageous aspects are included in the dependent claims.

In one aspect, the present disclosure provides a system for supporting tissue. The system includes two or more sensor assemblies, each of the sensor assemblies including a base portion configured to be anchored in tissue, a beam extending away from the base portion toward an attachment surface, and a sensor attached to the beam and configured to measure a force applied to the beam. The system further includes a mesh configured to be disposed adjacent to the tissue, the mesh configured to attach to the attachment surface of each of the sensor assemblies such that mesh tension applies a shear force to the attachment surface of at least one sensor assembly. The system further includes readout electronics configured to receive force information from the sensor of each sensor assembly.

Implementations of the disclosure may include one or more of the following optional features. In some examples, the sensor includes a strain gauge configured to measure the shear force applied to the attachment surface of the sensor assembly. The system may further include a second sensor attached to the beam, the second sensor configured to measure a second shear force applied to the attachment surface of the sensor assembly. The sensor and the second sensor may be disposed on the beam such that the sensors measure shear forces applied to the attachment surface in substantially orthogonal directions. In some examples, the sensor and the second sensor are disposed on the beam such that the sensors measure a direction of shear force applied to the attachment surface of the sensor assembly. The beam may be a cylinder. The beam may be configured to rotate with respect to the base portion. The system may further include a protective covering surrounding the beam. The mesh may be anisotropic. In some examples, the mesh is configured to attach to the respective attachment surface of each of the two or more sensor assemblies with sutures that extend through holes disposed in the attachment surface. The base portion may be configured to attach to the respective attachment surface of each of the two or more sensor assemblies with sutures that extend through holes disposed in the base portion and additional holes disposed in the attachment surface.

In another aspect, the disclosure provides a method for measuring shear forces applied by a surgical mesh. The method includes implanting two or more sensor assemblies by anchoring a base portion of each of the two or more sensor assemblies to tissue, such that a beam of each of the two or more sensor assemblies extends away from the base portion toward an attachment surface of each of the two or more sensor assemblies. The method further includes disposing a mesh adjacent to the tissue and securing the mesh to the respective attachment surface of each of the two or more sensor assemblies such that mesh tension applies a shear force to the attachment surface of at least one of the two or more sensor assemblies. The method further includes interfacing readout electronics to a sensor attached to the beam and configured to measure a force applied to the beam. The method further includes receiving, using the readout electronics, force information from each sensor assembly.

Implementations of the disclosure may include one or more of the following optional features. In some examples, receiving force information includes wirelessly receiving force information. Implanting two or more sensor assemblies may include implanting at least one sensor assembly having at least one strain gauge configured to measure the shear force applied to the attachment surface of the sensor assembly. Receiving force information may include receiving force information from the at least one strain gauge of the sensor assembly. In some examples, implanting two or more sensor assemblies includes implanting at least one sensor assembly having two strain gauges disposed on the beam such that the sensors measure shear forces applied to the attachment surface in substantially orthogonal directions. Receiving force information may include receiving force information from the two strain gauges of the sensor assembly. Receiving force information may include receiving force direction information. In some examples, securing the mesh to the respective attachment surface includes attaching the mesh to the respective attachment surface of each of the two or more sensor assemblies with sutures that extend through holes disposed in the attachment surface. Disposing a mesh adjacent to the tissue may include disposing an anisotropic mesh adjacent to the tissue.

In another aspect, the disclosure provides a sensor assembly. The sensor assembly includes a base portion configured to be anchored in tissue. The sensor assembly further includes a beam extending away from the base portion toward an attachment surface, the attachment surface configured to engage a mesh disposed adjacent to the tissue. The sensor assembly further includes a sensor attached to the beam and configured to measure a force applied to the beam by the mesh through the attachment surface. The sensor assembly further includes readout electronics configured to receive force information from the sensor.

### BRIEF DESCRIPTION OF THE DRAWINGS

The accompanying drawings are incorporated into this document and form a part of the specification.
Fig. 1 illustrates an example implant system.
Fig. 2 illustrates a side view of the example implant system.
Fig. 3 illustrates a close-up view of an example sensor assembly.
Figs. 4A and 4B are perspective views of portions of example sensor assemblies.
Fig. 5 illustrates another close-up view of an example sensor assembly.
Fig. 6 shows an example attachment of a mesh to a sensor assembly.
Fig. 7 is a flowchart of a method for measuring shear forces applied by a surgical mesh.

In the drawings, like reference numbers generally indicate identical or similar elements. Additionally, generally, the left-most digit(s) of a reference number identifies the drawing in which the reference number first appears.

### DETAILED DESCRIPTION

This document describes system, apparatus, device, and/or method embodiments, and/or combinations and sub-combinations of any of the above, for textile-based implants configured to measure shear forces at fixation points.

In some embodiments, as used in the specification and including the appended claims, the singular forms "a," "an," and "the" include the plural, and reference to a particular numerical value includes at least that particular value, unless the context clearly dictates otherwise. Ranges may be expressed herein as from "about" or "approximately" one particular value and/or to "about" or "approximately" another particular value. When such a range is expressed, another embodiment includes from the one particular value and/or to the other particular value. Similarly, when values are expressed as approximations, by use of the antecedent "about," it will be understood that the particular value forms another embodiment. It is also understood that all spatial references, such as, for example, horizontal, vertical, top, upper, lower, bottom, left and right, are for illustrative purposes only and can be varied within the scope of the disclosure. For example, the references "upper" and "lower" are relative and used only in the context to the other and are not necessarily "superior" and "inferior." Generally, similar spatial references of different aspects or components indicate similar spatial orientation and/or positioning, i.e., that each "first end" is situated on or directed towards the same end of the device.

It is to be appreciated that the Detailed Description section, and not any other section, is intended to be used to interpret the claims. Other sections can set forth one or more but not all exemplary embodiments as contemplated by the inventor(s), and thus, are not intended to limit this disclosure or the appended claims in any way.

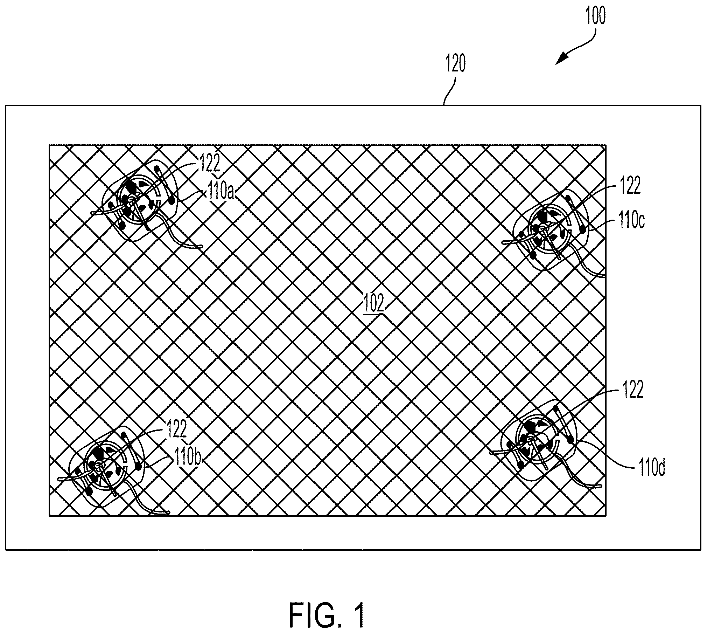

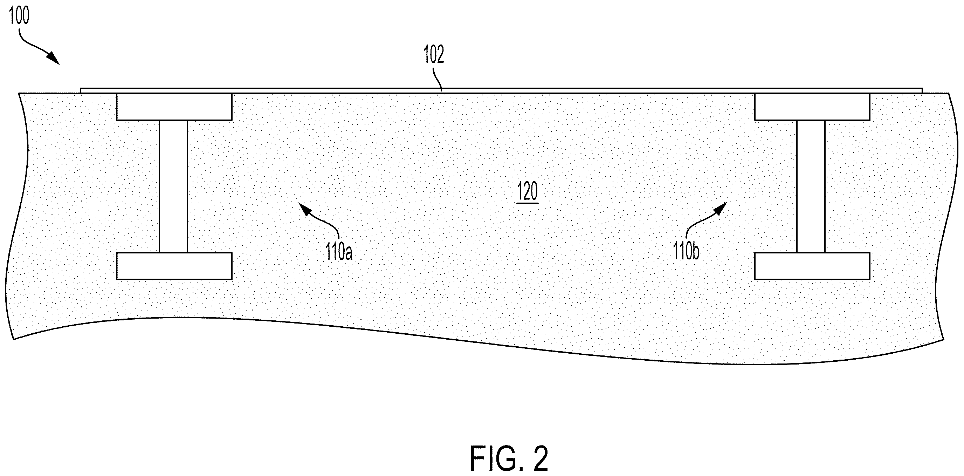

Referring to Fig. 1, an example implant system 100 is shown. The system 100 may be used to provide support to defective or compromised tissue 120 (Fig. 2) of, e.g., a human or animal. For example, the system 100 may be used in an intervention to address a hernia. The system 100 includes a flexible mesh 102 that may provide temporary or permanent structural support for the compromised tissue 120, nearby organs, or other tissues. In some examples, the mesh 102 is applied to a surface layer of tissue 120, such as an abdominal wall, such that the mesh 102 substantially conforms to the surface layer of tissue 120. The mesh 102 may include or be composed of synthetic and/or biological (e.g., animal-derived) materials. Synthetic materials may be absorbable, non-absorbable or a combination of absorbable and non-absorbable materials. Animal-derived meshes 102 may be made of animal tissue, such as intestine or skin, that has been processed and disinfected to be suitable for use as an implanted device. The mesh 102 may include woven and/or knitted materials and/or sheet materials. In some examples, the mesh 102 includes anisotropic properties. That is, physical properties of the mesh 102, such as stiffness, elasticity, yield strength, etc., may have a directional dependence. In other words, the mesh 102 may require more force to stretch in one direction than in another. In one example, a rectangular mesh 102 may require more force to stretch a distance transversely than is required to stretch the same distance longitudinally. These and other properties of the mesh 102 may be selected for compatibility with the tissue 120 being supported and/or repaired, e.g., similar elasticity.

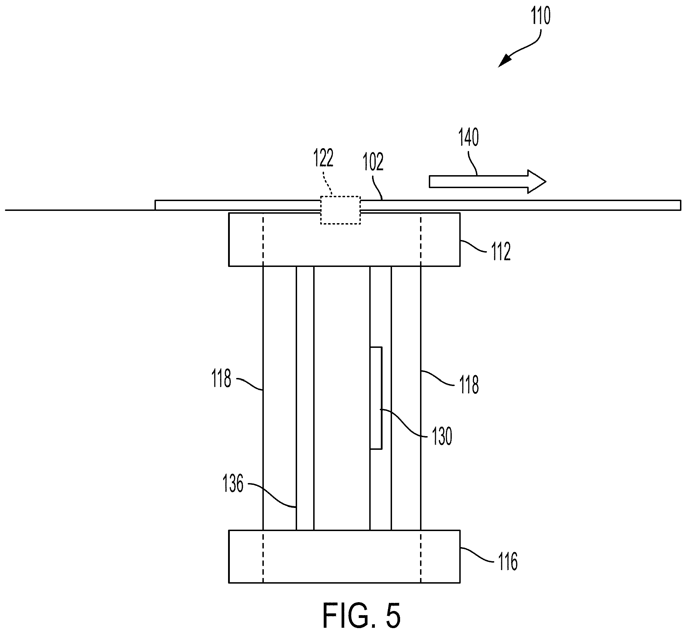

The mesh 102 may serve as the primary repair to the compromised tissue 120, or may strengthen a repair or reconstruction performed via another means. In either case, after the mesh 102 is implanted, it may be secured in place at multiple fixation points. For example, the mesh 102 may be tacked, stapled, or sutured directly to tissue 120 at the fixation points. While meshes 102 are generally flexible, tension in the mesh 102 will apply forces to the fixation points, generally in the form of shear forces 140 (Fig. 5). Unduly large forces may result in pain, tissue damage, increased risk of mesh failure, and/or hernia relapse. The force applied to each fixation point may be reduced by increasing the number of fixation points. However, increasing the number of fixation points may require longer surgical time, and/or limit the flexibility of the mesh 102, increasing pain and discomfort. Furthermore, merely increasing the number of fixation points may not entirely preclude undue forces at any one particular fixation point. As discussed in greater detail in the following paragraphs, an implant system 100 may include fixation points that include sensor assemblies 110 configured to measure and report shear forces 140. By providing post-operative indications of shear forces 140, undue forces may be detected early enough to address before, e.g., tissue damage occurs. This may give surgeons confidence to implant surgical meshes 102 having fewer fixation points, thus saving time and effort, while avoiding the aforementioned negative issues and outcomes.

As shown in Fig. 1, a rectangular mesh 102 is attached to four sensor assemblies 110a-110d. That is, each sensor assembly 110 also functions as an attachment point. As shown, the mesh 102 is attached to each sensor assembly 110 by suitable attachment means 122, such as sutures, tacks, staples, or the like. Each sensor assembly 110 may be embedded or anchored in position, e.g., at appropriate locations with respect to compromised tissue 120. After the sensor assemblies 110 are anchored in position, the mesh 102 may be applied over the compromised tissue 120 (and/or over a repair to the compromised tissue 120) and secured to the sensor assemblies 110. Referring to Fig. 2, a side view of the example implant system 100 is shown. The side view more clearly shows the relationship between the tissue 120, the mesh 102, and example sensor assemblies 110a and 1 10b. As shown, the sensor assemblies 110 are entirely embedded within the tissue 120, and the mesh 102 is adjacent to and conforms with the surface of the tissue 120. Due to the disclosed arrangement, longitudinal tension in the mesh 102 will result in a shear forces 140 at sensor assembly 110a in the direction of sensor assembly 110b and vice versa (and, similarly, between sensor assemblies 110c and 110d). Likewise, transverse tension in the mesh 102 will result in shear forces 140 at sensor assembly 110a in the direction of sensor assembly 110c and vice versa (and, similarly, between sensor assemblies 110b and 110d). By logical extension, the shear forces 140 applied by the mesh 102 to any and all sensor assemblies 110 depend on the geometric relationship between respective sensor assemblies 110, the tension in the mesh 102, physical properties of the mesh 102, and so forth. For example, an anisotropic mesh may preferentially apply forces in particular directions. Other arrangements of sensor assemblies 110 are also possible. In some examples, a portion of one or more assemblies may protrude beyond the surface of the tissue 120, e.g., to provide for an antenna and/or other access to readout electronics (discussed below) or other purposes.

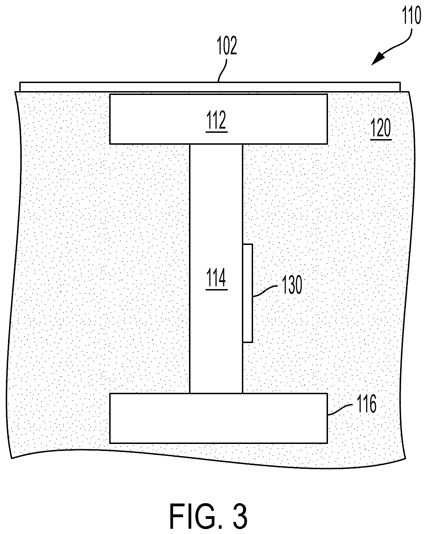

Referring to Fig. 3, a close-up view of one example sensor assembly 110 is shown. Each sensor assembly 110 may be constructed from stainless steel, Polyether ether ketone (PEEK), and/or other bio-compatible materials having well-understood physical properties. As shown, the sensor assembly 110 includes a base portion 116, a beam 114 extending away from the base portion 116 and toward the attachment surface 112, which defines the fixation point where the mesh 102 is attached to the sensor assembly 110. In some examples, the attachment surface 112 is a surface of the beam 114 or is a surface of a separate component which is affixed to the beam 114. That is, the mesh may be fixated to an upper surface of the beam 114. In some examples, the attachment surface 112 is a surface of a separate component which is free to rotate with respect to the beam 114 and/or slide along the beam 114, e.g., until attached to the mesh 102. In each of these example embodiments, force applied by the mesh 102 to the attachment surface 112 is transmitted to the beam 114. One or more sensors 130, such as force sensors 130 are attached to the beam 114 or otherwise configured to measure aspects of the beam 114, such as forces applied to the beam 114 and/or the mechanical response of the beam 114 to the applied forces, such as the amount of bending due to forces. Additional sensors 130 disposed on or adjacent to the beam 114 may measure additional aspects of the sensor assembly 110, such as temperature. The entire sensor assembly 110 may be compact so as to be less invasive and/or avoid interfering with the function of surrounding tissue 120 (e.g., muscle). Therefore, the beam 114 may be relatively compact in length while also being sufficiently long to provide appropriate measurement resolution. The measurement resolution may also depend on the cross section or other characteristics of the beam 114. In some embodiments, the beam 114 is between 5 and 20 mm long.

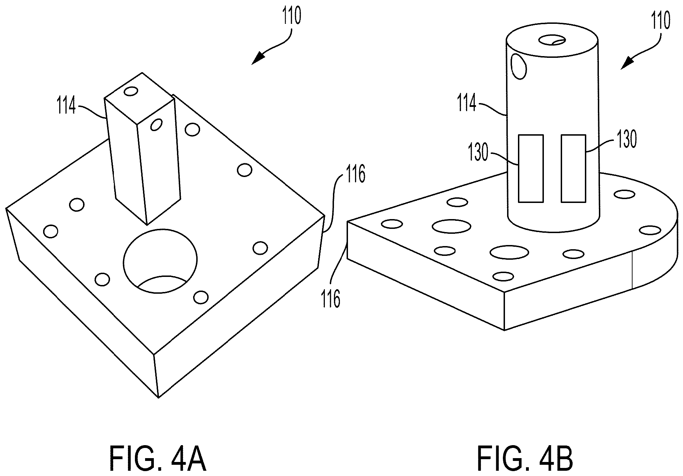

Figs. 4A and 4B show perspective views of portions of example sensor assemblies 110, including beams 114, base portions 116, and sensors 130. Referring back to Fig. 3, the base portion 116 is configured to be embedded in and/or anchored to surrounding tissue 120 (e.g., muscle). The base portion 116 may be constructed of a plate of material having sufficient surface area to provide a strong anchor. In some examples, the plate may be substantially solid so as to provide a rigid platform for supporting the beam 114. The base portion 116 may also include an arrangement of suture holes or other means for attaching the base portion 116 to the surrounding tissue 120. As discussed above, the beam 114 extends away from the base portion 116 toward an attachment surface 112 and fixation point of the mesh 102. Thus, shear forces 140 applied to the attachment surface 112 by tension in the mesh 102 are transferred to the beam 114 and the base portion 116. Because the base portion 116 provides a stable support for the beam 114, the shear force 140 may cause the beam 114 to deform relative to the base portion 116, e.g., to elastically bend in the direction of the force. One or more of the sensors 130 may be a strain gauge that is configured to measure the elastic deformation of the beam 114. The output of the strain gauge can then be used to determine the shear force 140 applied at the fixation point (and, therefore, the tension in the mesh 102) based on the geometry of the sensor assembly 110 and the material properties of the beam 114 and/or other components of the sensor assembly 110.

In some examples, the beam 114 is configured to preferentially bend in particular directions. For example, the beam 114 illustrated in Fig. 4A is rectangular and may preferentially bend towards one of its faces. In these examples, sensors 130 (e.g., strain gauges) may be disposed on one or more faces of the beam 114. In other examples (e.g., as illustrated in Fig. 4B), the beam 114 may be cylindrical and may not preferentially bend in any particular direction. In these examples, sensors 130 (e.g., strain gauges) may be disposed on a side of the beam 114 that faces a likely direction of shear forces 140, such as toward the center of the mesh 102. As shown, two adjacent sensors 130 are shown on the cylindrical beam 114 of Fig. 4B. Other beam shapes and properties may be used to enhance the sensing capabilities of the beam 114, including (but not limited to) prisms or frusto-conicals that are rectangular, square, cylindrical, etc. Using these or other shapes, shear forces 140 applied at the fixation point may be predictably translated into deformations of the beam 114 at the position of the sensor 130, such that the expected deformations are well matched to the sensing characteristics (e.g., range and resolution) of the sensors 130.

In some examples, a beam 114 may be configured to rotate or pivot with respect to the base portion 116, so that a sensor 130 disposed on the beam 114 may be aligned with the likely direction of a shear force 140. For example, the beam 114 may include a head or other structure that limits transverse movement of the beam 114 with respect to the base portion 116, while allowing the beam 114 to rotate. Other embodiments may include bearings, C-clips, or other rotation-allowing interfaces between the beam 114 and the base portion 116. In some examples, the beam 114 is manually rotated to an appropriate configuration as the sensor assembly is installed. In other examples, the forces applied by the mesh 102 cause the beam 114 to rotate automatically due to forces applied by the mesh 102. In some examples, sensors 130 may be disposed at several locations on the beam 114, e.g., on multiple faces of a multi-faceted beam 114, e.g., to measure shear forces 140 in several directions. In some examples, the measurements of the individual sensors 130 are combined to determine an aggregate direction and magnitude of the shear force 140. For example, the aggregate direction and magnitude may be determined as the vector sum of individually determined shear forces 140. In some examples, the material properties of the beam 114 may also be used to determine the overall shear force 140. For example, e.g., as discussed above, beams 114 may preferentially bend in particular directions. Thus, the amount of beam deformation in a preferred direction may be greater than other directions for the same magnitude of shear force 140. Thus, determining the aggregate direction and magnitude of the shear force 140 may also be based on the orientation and/or configuration of the beam 114 as well as material properties of the beam 114.

In some examples, the beam 114 may be surrounded by or encased within a protective covering (136, Fig. 5). The covering 136 may protect the beam 114 and/or sensor 130 from contacting surrounding tissue 120 and vice versa. The protective covering 136 may be constructed using the same material as the beam 114 or a different material. In some examples, the protective covering 136 is a coating applied to the beam 114. In some examples, the protective covering 136 is a separate structure that surrounds the beam 114. In some examples, the beam 114 is free to move with respect to (e.g., rotate within) the protective covering 136.

Referring to Fig. 5, another close-up view of an example sensor assembly 110 is shown. Fig. 5 shows connections between components of the sensor assembly 110 as well as an example method of attaching the mesh 102 to the attachment surface 112 of the sensor assembly 110. As shown, sutures 118 run through holes in the base portion 116 and through corresponding holes in the attachment surface 112 to maintain the base portion's 116 position with respect to the tissue 120. The attachment surface is then secured to the mesh 102 by an attachment means 122. The attachment means 122 may be sutures, tacks, staples, or other means that securely attaches the mesh 102 to the attachment surface 112. Thus, tension in the mesh 102 is transferred to the attachment surface 112 of the sensor assembly 110 as a shear force 140, as indicated by the arrow. Other techniques of attaching the mesh 102 to the attachment surface 112 of the sensor assembly 110 are also possible, including directly suturing, stapling, or tacking the mesh 102 to the attachment surface 112 of the sensor assembly 110. In some examples, the mesh 102 is secured directly to the beam 114. In these examples, sutures 118 may run through holes in the base portion 116 to an upper portion (similar in configuration to the illustrated attachment surface 112) to better secure the base portion 116 to tissue 120. In some examples, the sutures run through tissue/muscle, some portion of which is "sandwiched" between the base portion 116 and the upper portion.

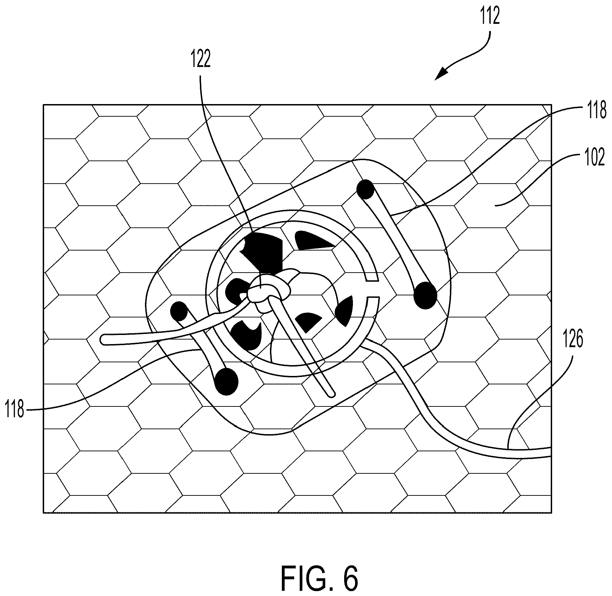

Fig. 6 shows an example attachment of a mesh 102 to the attachment surface 112 of a sensor assembly 110. As shown, the mesh is attached using sutures as the attachment means 122. These attachment sutures are separate from the sutures 118 that secure the base portion 116 to the tissue 120. The sensors 130 interface with readout electronics (not shown) using a wired connection 126 protruding from the sensor assembly 110, e.g., at or adjacent to the attachment surface 112. In other embodiments, the sensors 130 interface with readout electronics using a wireless connection. In wireless embodiments, an antenna may be disposed adjacent to the attachment surface 112 or other location that reduces the distance from the antenna to the readout electronics. In some examples, the sensor assembly 110 includes circuitry, power source (e.g., inductively rechargeable battery), and memory for recording data from the sensors 130. The recorded data may be uploaded to readout electronics (e.g., along with recorded timestamps associated with recorded data points) when the sensor assembly 110 is connected to, or comes within wireless range of, the readout electronics. In some examples, the data is read out during scheduled post-operative sessions and/or while performing post-operative protocols. In this way, the post-operative forces applied by the mesh 102 may be monitored to detect off-normal situations.

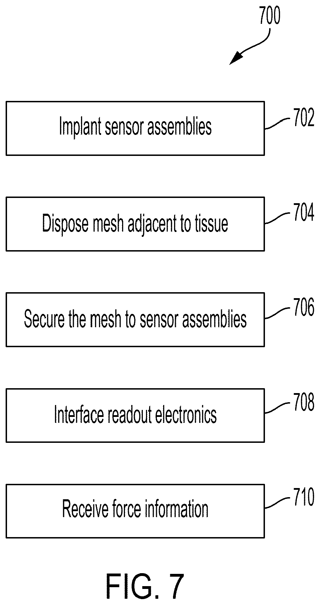

Referring to Fig. 7, a flowchart 700 of a method for measuring shear forces 140 applied by a surgical mesh 102 is shown. At step 702, the method includes implanting two or more sensor assemblies 110 by anchoring a base portion 116 of each of the two or more sensor assemblies 110 to tissue 120, such that a beam 114 of each of the two or more sensor assemblies 110 extends away from the base portion 116 toward an attachment surface 112 of each of the two or more sensor assemblies 110. As described above, anchoring the base portion 116 may include embedding the sensor assembly 110 within tissue 120 (e.g., muscle) and securing the base portion 116 to the tissue 120, e.g., using sutures 118. At step 704, the method includes disposing a mesh 102 adjacent to the tissue 120. As described above, the mesh 102 may be in a sheet or woven/knitted form and configured to provide support, e.g., to defective, damaged, or compromised tissue 120. The mesh 102 may be disposed so as to conform to a surface or boundary layer of the tissue 120, e.g., overlaying the compromised portion of the issue. For example, the mesh 102 may be disposed to overlay a damaged portion of an abdominal wall.

At step 706, the method includes securing the mesh 102 to the respective attachment surface 112 of each of the two or more sensor assemblies 110 such that when the mesh is in tension, it applies a shear force 140 to the attachment surface 112 of at least one of the two or more sensor assemblies 110. That is, the mesh may be secured under tension, such that changes in the tension can be sensed by the sensor assemblies 110. Alternatively, the mesh may be secured stress-free, allowing the sensor assemblies 110 to sense forces cause by, e.g., intra-abdominal forces applied to the entire system after fixation. As described above, attaching the mesh 102 may include suturing the mesh 102 to the attachment surface 112. At step 708, the method includes interfacing readout electronics to a sensor 130 attached to the beam 114 and configured to measure a force applied to the beam 114. At step 710, the method includes receiving, using the readout electronics, force information from the sensor 130 of each sensor assembly 110.

After the force information is received, it may be used for a variety of purposes. For example, the information could be used in ex vivo studies to evaluate the impact of the mesh pattern on the force at where the mesh is secured. The information may also be used to optimize mesh design by comparing information from meshes constructed from various materials or combinations of materials, and/or having particular dimensions or configurations. Such information may be used to demonstrate regulatory compliance and/or demonstrate superiority of a mesh supplier's products over competing mesh products. The information could also be used post-implantation in patients to highlight safety issues, such as imminent or actual mesh failure, or undue forces leading to pain or discomfort.

While this disclosure describes example embodiments for example fields and applications, it should be understood that the disclosure is not limited to the disclosed examples. Other embodiments and modifications thereto are possible, and are within the scope and spirit of this disclosure. For example, and without limiting the generality of this paragraph, embodiments are not limited to the software, hardware, firmware, and/or entities illustrated in the figures and/or described in this document. Furthermore, embodiments (whether or not explicitly described) have significant utility to fields and applications beyond the examples described in this document.

Embodiments have been described in this document with the aid of functional building blocks illustrating the implementation of specified functions and relationships. The boundaries of these functional building blocks have been arbitrarily defined in this document for the convenience of the description. Alternate boundaries can be defined as long as the specified functions and relationships (or their equivalents) are appropriately performed. Also, alternative embodiments can perform functional blocks, steps, operations, methods, etc. using orderings different than those described in in this document.

The features from different embodiments disclosed herein may be freely combined. For example, one or more features from a method embodiment may be combined with any of the system or product embodiments. Similarly, features from a system or product embodiment may be combined with any of the method embodiments herein disclosed.

References in this document to "one embodiment," "an embodiment," "an example embodiment," or similar phrases, indicate that the embodiment described can include a particular feature, structure, or characteristic, but every embodiment can not necessarily include the particular feature, structure, or characteristic. Moreover, such phrases are not necessarily referring to the same embodiment. Furthermore, when a particular feature, structure, or characteristic is described in connection with an embodiment, it would be within the knowledge of persons skilled in the relevant art(s) to incorporate such feature, structure, or characteristic into other embodiments whether or not explicitly mentioned or described in this document. Additionally, some embodiments can be described using the expression "coupled" and "connected" along with their derivatives. These terms are not necessarily intended as synonyms for each other. For example, some embodiments can be described using the terms "connected" and/or "coupled" to indicate that two or more elements are in direct physical or electrical contact with each other. The term "coupled," however, can also mean that two or more elements are not in direct contact with each other, but still co-operate or interact with each other.

The breadth and scope of this disclosure should not be limited by any of the above-described example embodiments, but should be defined only in accordance with the following claims and their equivalents.

The invention may further be described by reference to the following clauses:
Clause 1: A system for supporting tissue, the system comprising:
   two or more sensor assemblies, each of the two or more sensor assemblies comprising:
      a base portion configured to be anchored in tissue;
      a beam extending away from the base portion toward an attachment surface; and
      a sensor attached to the beam and configured to measure a force applied to the beam;
   a mesh configured to be disposed adjacent to the tissue, the mesh configured to attach to the respective attachment surface of each of the two or more sensor assemblies such that mesh tension applies a shear force to the attachment surface of at least one of the two or more sensor assemblies; and
   readout electronics configured to receive force information from the sensor of each sensor assembly.
Clause 2: The system of clause 1, wherein the sensor comprises a strain gauge configured to measure the shear force applied to the attachment surface of the sensor assembly.
Clause 3. The system of clause 2, further comprising a second sensor attached to the beam, the second sensor configured to measure a second shear force applied to the attachment surface of the sensor assembly.
Clause 4. The system of clause 3, wherein the sensor and the second sensor are disposed on the beam such that the sensors measure shear forces applied to the attachment surface in substantially orthogonal directions.
Clause 5. The system of clause 3 or 4, wherein the sensor and the second sensor are disposed on the beam such that the sensors measure a direction of shear force applied to the attachment surface of the sensor assembly.
Clause 6. The system of any preceding clause, wherein the beam is a cylinder.
Clause 7. The system of any preceding clause, wherein the beam is configured to rotate with respect to the base portion.
Clause 8. The system of any preceding clause, further comprising a protective covering surrounding the beam.
Clause 9. The system of any preceding clause, wherein the mesh is anisotropic.
Clause 10. The system of any preceding clause, wherein the mesh is configured to attach to the respective attachment surface of each of the two or more sensor assemblies with sutures that extend through holes disposed in the attachment surface.
Clause 11. The system of any preceding clause, wherein the base portion is configured to attach to the respective attachment surface of each of the two or more sensor assemblies with sutures that extend through holes disposed in the base portion and additional holes disposed in the attachment surface.
Clause 12. A method for measuring shear forces applied by a surgical mesh, the method comprising:
   implanting two or more sensor assemblies by anchoring a base portion of each of the two or more sensor assemblies to tissue, such that a beam of each of the two or more sensor assemblies extends away from the base portion toward an attachment surface of each of the two or more sensor assemblies;
   disposing a mesh adjacent to the tissue;
   securing the mesh to the respective attachment surface of each of the two or more sensor assemblies such that mesh tension applies a shear force to the attachment surface of at least one of the two or more sensor assemblies;
   interfacing readout electronics to a sensor attached to the beam and configured to measure a force applied to the beam; and
   receiving, using the readout electronics, force information from each sensor assembly.
Clause 13. The method of clause 12, wherein receiving force information comprises wirelessly receiving force information.
Clause 14. The method of clause 12 or 13, wherein implanting the two or more sensor assemblies comprises implanting at least one sensor assembly comprising at least one strain gauge configured to measure the shear force applied to the attachment surface of the sensor assembly.
Clause 15. The method of clause 14, wherein receiving force information comprises receiving force information from the at least one strain gauge of the sensor assembly.
Clause 16. The method of any of clauses 12-15, wherein implanting the two or more sensor assemblies comprises implanting at least one sensor assembly comprising two strain gauges disposed on the beam such that the sensors measure shear forces applied to the attachment surface in substantially orthogonal directions.
Clause 17. The method of clause 16, wherein receiving force information comprises receiving force information from the two strain gauges of the sensor assembly.
Clause 18. The method of clause 17, wherein receiving force information comprises receiving force direction information.
Clause 19. The method of any of clauses 12-18, wherein securing the mesh to the respective attachment surface comprises attaching the mesh to the respective attachment surface of each of the two or more sensor assemblies with sutures that extend through holes disposed in the attachment surface.
Clause 20. The method of any of clauses 12-19, wherein disposing a mesh adjacent to the tissue comprises disposing an anisotropic mesh adjacent to the tissue.
Clause 21. A sensor assembly comprising:
   a base portion configured to be anchored in tissue;
   a beam extending away from the base portion toward an attachment surface, the attachment surface configured to engage a mesh disposed adjacent to the tissue;
   at least two sensors attached to the beam and configured to measure a force applied to the beam by the mesh through the attachment surface; and
   readout electronics configured to receive force information from the at least two sensors.

## Claims

1. A system for supporting tissue, the system comprising:
two or more sensor assemblies (110), each of the two or more sensor assemblies (110) comprising:
a base portion (116) configured to be anchored in tissue (120);
a beam (114) extending away from the base portion (116) toward an attachment surface (112); and
a sensor (130) attached to the beam (114) and configured to measure a force applied to the beam (114);
a mesh (102) configured to be disposed adjacent to the tissue (120), the mesh (102) configured to attach to the respective attachment surface (112) of each of the two or more sensor assemblies (110) such that mesh tension applies a shear force (140) to the attachment surface (112) of at least one of the two or more sensor assemblies (110); and
readout electronics configured to receive force information from the sensor (130) of each sensor assembly (110).

2. The system of claim 1, wherein the sensor (130) comprises a strain gauge configured to measure the shear force (140) applied to the attachment surface (112) of the sensor assembly (110).

3. The system of claim 2, further comprising a second sensor (130) attached to the beam (114), the second sensor (130) configured to measure a second shear force (140) applied to the attachment surface (112) of the sensor assembly (110).

4. The system of claim 3, wherein the sensor (130) and the second sensor (130) are disposed on the beam (114) such that the sensors (130) measure shear forces (140) applied to the attachment surface (112) in substantially orthogonal directions.

5. The system of claim 3 or 4, wherein the sensor (130) and the second sensor (130) are disposed on the beam (114) such that the collective sensor (130) measurements indicate a direction of shear force (140) applied to the attachment surface (112) of the sensor assembly (110).

6. The system of any of claims 1-5, wherein the beam (114) is a cylinder.

7. The system of any of claims 1-6, wherein the beam (114) is configured to rotate with respect to the base portion (116).

8. The system of any of claims 1-7, further comprising a protective covering surrounding the beam (114).

9. The system of any of claims 1-8, wherein the mesh (102) is anisotropic.

10. The system of any of claims 1-9, wherein the mesh (102) is configured to attach to the respective attachment surface (112) of each of the two or more sensor assemblies (110) with sutures (118) that extend through holes disposed in the attachment surface (112).

11. The system of any of claims 1-10, wherein the base portion (116) is configured to attach to the respective attachment surface (112) of each of the two or more sensor assemblies (110) with sutures (118) that extend through holes disposed in the base portion (116) and additional holes disposed in the attachment surface (112).

12. A method for measuring shear forces applied by a surgical mesh (102), the method comprising:
implanting two or more sensor assemblies (110) by anchoring a base portion (116) of each of the two or more sensor assemblies (110) to tissue (120), such that a beam (114) of each of the two or more sensor assemblies (110) extends away from the base portion (116) toward an attachment surface (112) of each of the two or more sensor assemblies (110);
disposing a mesh (102) adjacent to the tissue (120);
securing the mesh (102) to the respective attachment surface (112) of each of the two or more sensor assemblies (110) such that mesh tension applies a shear force (140) to the attachment surface (112) of at least one of the two or more sensor assemblies (110);
interfacing readout electronics to a sensor (130) attached to the beam (114) and configured to measure a force applied to the beam (114); and
receiving, using the readout electronics, force information from each sensor assembly (110).

13. The method of claim 12, wherein receiving force information comprises wirelessly receiving force information.

14. The method of claim 12 or 13, wherein implanting two or more sensor assemblies (110) comprises implanting at least one sensor assembly (110) comprising at least one strain gauge configured to measure the shear force (140) applied to the attachment surface (112) of the sensor assembly (110).

15. The method of claim 14, wherein receiving force information comprises receiving force information from the at least one strain gauge of the sensor assembly (110).
